Europäisches Patentamt

European Patent Office

Office européen des brevets

(19)

(11) **EP 1 547 587 A1**

(12) **EUROPEAN PATENT APPLICATION**
published in accordance with Art. 158(3) EPC

(43) Date of publication:
**29.06.2005 Bulletin 2005/26**

(21) Application number: **03792765.4**

(22) Date of filing: **21.08.2003**

(51) Int Cl.[7]: **A61K 31/196**, A61K 31/5575,
A61K 9/48, A61K 47/14,
A61K 47/38, A61P 1/00,
A61P 13/12, A61P 29/00,
A61P 43/00

(86) International application number:
**PCT/JP2003/010562**

(87) International publication number:
**WO 2004/017954 (04.03.2004 Gazette 2004/10)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **22.08.2002 JP 2002241271**

(71) Applicant: **ONO PHARMACEUTICAL CO., LTD.
Osaka-shi, Osaka 541-8526 (JP)**

(72) Inventors:
• **SAKAI, Yoshiki, Ono Pharmaceutical Co., Ltd.
Mishima-gun, Osaka 618-8585 (JP)**

• **KATSUBE, Nobuo, Ono Pharmaceutical Co., Ltd.
Sakai-gun, Fukui 913-0032 (JP)**
• **NISHIURA, Akio, Ono Pharmaceutical Co., Ltd.
Osaka 618-8585 (JP)**
• **YAMAMOTO, Masanobu,
Ono Pharmaceutical Co., Ltd.
Mishima-gun, Osaka 618-8585 (JP)**
• **SUGISHITA, Ken-ichi,
Ono Pharmaceutical Co., Ltd.
Mishima-gun, Osaka 618-8585 (JP)**

(74) Representative: **Henkel, Feiler & Hänzel
Möhlstrasse 37
81675 München (DE)**

(54) **AGENT FOR REDUCING SIDE EFFECTS OF DICLOFENAC**

(57)   The present invention relates to reduction of side effects of diclofenac or a salt thereof. An agent for reducing side effects of diclofenac or a salt thereof which comprises ornoprostil. It is expected that a combination agent of diclofenac or a nontoxic salt thereof and ornoprostil is comparable to even superior to marketed diclofenac tablets or Arthrotec tables in effects and fast-acting property while showing little side effects (particularly digestive disorders, gastric ulcer, diarrhea/vomiting and renal disorder) and exerting excellent antipyretic, analgesic and antiinlfammatory effects. Also, by formulating the combination agent into a preparation of separation type pharmaceutical preparation, the stability of ornoprostil can be improved.

EP 1 547 587 A1

**Description**

Technical Field

The present invention relates to an agent for reducing side effects of diclofenac or a salt thereof, which comprises ornoprostil, and relates to a combination agent which comprises effective amounts of both components in one pharmaceutical preparation.

Background of the Invention

It is conventionally known that non-steroidal anti-inflammatory agents (non-steroidal anti-inflammatory drug; hereinafter referred to as "NSAID") are effective as antipyretic, analgesic and antiphlogistic agents, and a large number of compounds have been on the market. Particularly, NSAID have an increasing opportunity of being administered for a prolonged period of time as an antipyretic, an analgesic and an anti-inflammatory agent for febrile disease, collagen disease, plastic surgery-related disease and the like, and as a circulatory organ system disease-preventing agent for cerebrovascular accidents, ischemic heart disease and the like, and their using frequency is increasing more and more with the arrival of an aging society. It is said that these function mechanisms of NSAID are to inhibit cyclooxigenase enzyme (COX) of the synthesis pathway of prostaglandin (PG) $H_2$ from arachidonic acid, and thereby to control biosynthesis of various PG derivatives (e.g., $PGE_2$, $PGI_2$, $PGF_{2\alpha}$, $TXA_2$, $PGD_2$, *etc.*). It was formerly known that a constitutive type COX-1 and an inducible type COX-2 are present in COX, and it has been proposed recently that a constitutive type COX-3 is present, and a constitutive type is also present in COX-2 (*ProNAS, 99:* 13371, 2002). Conventionally, diclofenac, indometacin, acemetacin, aspirin, loxoprofen, ibuprofen and the like are known as typical examples of NSAID, but these have differences in terms of COX-1, COX-2 and COX-3 inhibitory activities, and these are characteristics of various NSAID For example, aspirin inhibits COX-1 and COX-3, but its COX-2 inhibitory activity is weak. Also, acetaminophen has been found as a compound which selectively inhibits COX-3. On the other hand, indometacin and diclofenac inhibit all of COX-1, COX-2 and COX-3, and diclofenac has the most strong inhibitory strength among conventionally known NSAID (*ProNAS, 99:* 13926, 2002).

Conventionally, it has been considered that NSAID causes digestive disorders such as gastric mucosal disorder, gastric ulcer, duodenal ulcer, and stomach region discomfort (hereinafter referred to as "digestive disorders"), because it inhibits COX-1 which carries out biosynthesis of PG (said to be mainly $PGI_2$ and $PGE_2$) that have the activity to protect the mucous membrane in digestive organs. Based on this consideration, a large number of COX-2 inhibitors have been developed as analgesic and antiphlogistic agents having less digestive disorders. For example, these include selecoxib, rofecoxib, meloxicam and the like (*ProNAS, 96*: 7563, 1999). However, it has been proved recently that COX-2 in the gastrointestinal organ mucous membrane is concerned in the ulcer restoration (*Gastroenterology, 112*: 387-397, 1997), and pointed out that a selective COX-2 inhibitor prolongs healing of an ulcerative lesion in which a mucous membrane disorder was generated due to *H. pylori* (*Helicobacter pylori*), acid in the stomach, ethanol and the like and inhibition of remaining COX-1. Accordingly, it has been suggested that onset of digestive disorders induced by NSAID requires inhibition of both COX-1 and COX-2 (*Gastroenterology, 119:* 706, 2000). In fact, in the case of selective COX-2 inhibitors such as selecoxib and rofecoxib, frequency of the onset of digestive disorders is low in comparison with the conventional nonselective COX inhibitor (NSAID), but the onset is certainly occurring *(Gastroenterology, 117*: 776, 1999, *Lancet, 354* (9196): 2106, 1999). Also, similar to the case of conventional NSAID, the selective COX-2 inhibitors have serious side effects of worsening nephropathy, because they reduce renal blood flow by inhibiting biosynthesis of PG (said to be mainly $PGI_2$ and $PGE_2$) in the kidney *(JPET, 298:* 735, 1999). In addition, there is a report that the generation frequency of ischemic heart disease is higher by a selective COX-2 inhibitor than by the conventional NSAID when administered for a prolonged period of time (*JPET, 289:* 735, 1999, *JAMA, 286*: 965, 2001). Digestive disorders by NSAID are considered to be side effects having the most high frequency among a large number of agents, and for example, according to epidemiology examination (*Rheumatism, 31*(1): 96-111, 1991) carried out by Japan Rheumatism Foundation, certain digestive disorders were found by endoscopic observation in 62.2% of the 1,008 cases of articular rheumatism patients who were taking NSAID. Particularly problematic in view of the treatment is that 41% thereof showed no symptoms.

On the other hand, PG is a physiologically active substance secreted from various tissues of the living body. Its activities cover broad ranges such as blood flow increasing activity, platelet agglutination inhibition activity, gastric acid secretion inhibition activity, gastric mucosa protection activity (site protection activity), uterine contraction activity and the like, and some of the PG derivatives are already on the market as antiulcer agents (e.g., ornoprostil, misoprostol, enprostil, *etc.*).

Up to now, attempts have been made to use PG derivatives with the aim of reducing the digestive disorders possessed by NSAID. For example, it has been reported that digestive disorders were reduced when indometacin and ornoprostil, or acemetacin and ornoprostil, were jointly used, in comparison with the single administration of NSAID (*Progress in Medicine, 9(7):* 183-190, 1989). Specification of WO 02/066030 describes a combination of dielofenac sodium and ornoprostil. Also, it has been reported that when effect of misoprostol on NSAID-induced gastric ulcer was examined, onset of gastric ulcer was inhibited, but various side effects on the digestive organ system were simultaneously and frequently generated, particularly with a high frequency of diarrhea (*Ann Intern Med, 119*(4): 257, 1993).

In addition, a combination agent containing diclofenac sodium and misoprostol (Arthrotec Tablet: trade name) has recently been developed, and is already on the market in Europe and America. However, such a combination of an NSAID with a PG derivative cannot said to be a sufficiently satisfactory combination, because its antipyretic, analgesic or antiphlogistic activity or its activity to reduce digestive disorders is not sufficient and it generates side effects of PG (diarrhea, abdominal pain, abdominal distension, indigestion, nausea, vomiting, *etc.*). For example, in the case of the administration of the above-described combination agent containing diclofenac sodium and misoprostol, instructions attached to the above combination agent describes that side effects of PG (diarrhea, loose passage, abdominal pain, abdominal distension, anorexia, indigestion, nausea, retching, vomiting, *etc.*) occurred frequently in comparison with the case of the single administration of diclofenac sodium. In addition, this combination agent is a two layer preparation containing misoprostol in the outer layer, and a diclofenac sodium preparation of an enteric state in the inner layer, but since diclafenac sodium is an enteric preparation, this has no immediate effect in terms of its analgesic activity.

Therefore, regarding the combination of diclofenac or a salt thereof having the strongest COX-1, COX-2 and COX-3 inhibitory activities with a PG derivative, great concern has been directed toward a novel combination which can keep effects of diclofenac or a salt thereof, can reduce side effects, such as digestive disorder and nephropathy, more effectively, and also can reduce side effects of PG derivatives, such as diarrhea and vomiting. Also, separate taking of diclofenac or a salt thereof and a PG derivative charges patients with burdens, such as forgetful of taking, too large or too small taking of one of them, so that great concern has been directed toward the development of a combination agent which can be taken as a single preparation, for the purpose of reducing the burdens to patients. Also, when diclofenac or a salt thereof and a PG derivative are contained in a single preparation, there is a possibility of spoiling stability of the PG derivative, so that designing of a combination agent in which the PG derivative is stable is also in demand. In addition, development of a more small-sized combination agent is also in demand for the purpose of improving compliance of patients in taking the combination agent.

Disclosure of the Invention

Accordingly, the object of the present invention is to provide a novel combination agent which can reduce side effects of diclofenac or a salt thereof and a PG derivative and in which the PG derivative is stable.

Taking the above-described problems into consideration, the present inventors have conducted intensive studies and found as a result that combination of diclofenac or a salt thereof and ornoprostil can keep the effects of diclofenac or a salt thereof and also can reduce side effects of diclofenac or a salt thereof, such as digestive disorder and nephropathy, and that ornoprostil in a combination agent containing diclofenac or a salt thereof and ornoprostil becomes considerably stable by carrying out the preparation designing shown below in such a manner that both components do not contact with each other, thereby accomplishing the present invention. In addition, it was found that miniaturization of the combination agent becomes possible while maintaining dissolution property and stability, by combining small seamless capsules containing ornoprostil with granules comprising diclofenac or a salt thereof and an excipient, thereby accomplishing the present invention.

That is, the present invention relates to the followings:

(1) An agent for reducing side effects of diclofenac or a salt thereof, which comprises ornoprostil as the active ingredient.

(2) The agent for reducing side effects according to (1), wherein the side effects are digestive disorders.

(3) The agent for reducing side effects according to (1), which is a combination agent comprising an effective amount of ornoprostil and an effective amount of diclofenac or a salt thereof.

(4) The agent for reducing side effects according to (3), wherein the effective amount of ornoprostil is a biological catalytic amount.

(5) The agent for reducing side effects according to (4), wherein the biological catalytic amount of ornoprostil is from 1/100,000 to 1/1,000 based on 1 part by weight of diclofenac or a salt thereof.

(6) The agent for reducing side effects according to (3), which comprises about 25 mg of diclofenac sodium and about 5 μg of ornoprostil.

(7) The agent for reducing side effects according to (3), which is a single solid preparation which comprises at least one small soft capsule preparation (A) comprising ornoprostil and an oily solution base, and a pharmaceutical composition (D-1) comprising diclofenac or a salt thereof and an excipient.

(8) The agent for reducing side effects according to (7), wherein the solid preparation is a hard capsule preparation.

(9) The agent for reducing side effects according to (8), which comprises from 40 to 95% by weight of the oily solution base based on 100% by weight of the small soft capsule preparation (A), and from 20 to 95% by weight of the excipient based on 100% by weight of the pharmaceutical composition (D-1).

(10) The agent for reducing side effects according to (8), wherein the number of the small soft capsule preparation (A) is from 1 to 400.

(11) The agent for reducing side effects according to (8), wherein the number of the small soft capsule preparation (A) is 1 or 2.

(12) The agent for reducing side effects according to (10), wherein the small soft capsule preparation (A) has an outer diameter of from 0.1 mm to 2 mm.

(13) The agent for reducing side effects according to (11), wherein the small soft capsule preparation (A) has an outer diameter of from 2 mm to 6 mm.

(14) The agent for reducing side effects according to (7), wherein the oily solution base is middle chain fatty acid triglyceride.

(15) The agent for reducing side effects according to (14), wherein the middle chain fatty acid triglyceride is tricaprylin.

(16) The agent for reducing side effects according to (7), wherein the excipient is one or at least two substances selected from the group consisting of saccharides, corn starch, and crystalline cellulose.

(17) The agent for reducing side effects according to (7), wherein the pharmaceutical composition (D-1) is in the form of granules.

(18) The agent for reducing side effects according to (17), wherein the granules have an average particle size of from about 200 μm to about 600 μm.

(19) The agent for reducing side effects according to (7), wherein the solid preparation is in the form of tablets.

(20) The agent for reducing side effects according to (3), which is a single solid preparation which comprises at least one small soft capsule preparation (A) comprising ornoprostil and an oily solution base, and at least one small soft capsule preparation (C) comprising diclofenac or a salt thereof and an oily solution base.

(21) The agent for reducing side effects according to (20), wherein the solid preparation is in the form of hard capsules.

(22) The agent for reducing side effects according to (20), wherein the oily solution base in the soft capsule preparation (A) is tricaprylin, and the oily solution base in the soft capsule preparation (C) is middle chain fatty acid triglyceride.

(23) The agent for reducing side effects according to (20), wherein the solid preparation is in the form of tablets.

(24) The agent for reducing side effects according to (3), which is a single solid preparation which comprises a pharmaceutical composition (B) comprising ornoprostil and an oily solution base, and at least one small soft capsule preparation (C) comprising diclofenac or a salt thereof and an oily solution base.

(25) The agent for reducing side effects according to (24), wherein the solid preparation is in the form of hard capsules.

(26) The agent for reducing side effects according to (24), wherein the solid preparation is in the form of soft capsules.

(27) The agent for reducing side effects according to (3), which is a single solid preparation which comprises at least one small soft capsule preparation (A) comprising ornoprostil and an oily solution base, and a pharmaceutical composition (D-2) comprising diclofenac or a salt thereof and an oily solution base.

(28) The agent for reducing side effects according to (27), wherein the solid preparation is in the form of soft capsules.

(29) The agent for reducing side effects according to (27), wherein the solid preparation is in the form of hard capsules.

(30) The agent for reducing side effects according to (3), which is a soft capsule preparation wherein a pharmaceutical composition (B) comprising the ornoprostil and an oily solution base is coated with a soft capsule coat comprising the diclofenac or a salt thereof

(31) The agent for reducing side effects according to (3), which is a soft capsule preparation wherein a pharmaceutical composition (D-2) comprising the diclofenac or a salt thereof and an oily solution base is coated with a soft capsule coat comprising the ornoprostil.

(32) A side effect-reducing hard capsule agent for reducing digestive disorders of diclofenac or a salt thereof, which is a hard capsule preparation which comprises at least one small soft capsule preparation (A-1) comprising ornoprostil and from 40 to 95% by weight of an oily solution base, and a pharmaceutical preparation (D-3) comprising diclofenac or a salt thereof and from 20 to 95% by weight of an excipient.

(33) The hard capsule agent for reducing side effects according to (32), wherein the oily solution base is tricaprylin, and the excipient is one or at least two substances selected from the group consisting of saccharides, corn starch, and crystalline cellulose.

According to the present invention, diclofenac is {2-[(2,6-dichlorophenyl)amino]phenyl}acetic acid, and all of pharmaceutically acceptable salts are included in the salt of diclofenac.

As the pharmaceutically acceptable salts, those which have no toxicity and are soluble in water are preferable. Examples of suitable salts include alkali metal (e.g., potassium, sodium, lithium, *etc.*) salts, alkaline earth metal (e.g., calcium, magnesium, *etc.*) salts, ammonium salts (e.g., tetramethylammonium salt, tetrabutylammonium salt, *etc.*), organic amine (e.g., triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, *etc.*) salts, acid addition salts (for example, inorganic acid salts (e.g., hydrochloride, hydrobromide, hydroiodide, sulfate, phosphate, nitrate, *etc.*), organic acid salts (e.g., acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, *etc.*), or the like.), or mixtures thereof. Also, the salts of the compound of the present invention include solvates, or solvates of alkali (earth) metal salts, ammonium salts, organic amine salts and acid addition salts of the above-described compound of the present invention. It is preferred that the solvates are nontoxic and soluble in water. Examples of suitable solvate include solvates of water, alcohol solvent (ethanol, *etc.*) and the like. The compound of the present invention is converted into a pharmaceutically acceptable salt by a conventionally known method.

Preferred as the salt of diclofenac is a sodium salt (diclofenac sodium).

The ornoprostil to be used in the present invention includes ornoprostil itself (17S,20-dimethyl-6-oxo-prostaglandin E1 methyl ester) or its α-, β-, or γ-cyclodextrinclathrate, and preferred is the ornoprostil itself

Since the pharmaceutical preparation of the present invention contains diclofenac or a salt thereof, it has antipyretic, analgesic and antiphlogistic activities and can be used as a therapeutic and/or preventive agent for, for example, articular rheumatism, rheumatic fever, osteoarthritis, tetanic spondylitis, spondylosis deformans, periarthritis (e.g., scapulohumeral periarthritis, etc.), cervico-omo-brachial syndrome, tendon marrow inflammation, neuralgia, joint pain, muscle pain, low back pain, pain or inflammation after operation, injury or tooth extraction, sprain pain, contusion pain, toothache, headache, pain of gout, menstrual pain, dysmenorrhea, after labor pain, pelvic inflammation, tergum inflammation, cystitis, anterior chamber inflammation and/or acute upper respiratory inflammation (e.g., common cold, cold syndrome, pharyngitis, laryngitis, *etc.*) and the like.

Appearance of digestive disorder and worsening of nephropathy can be sharply controlled without attenuating efficacy, by hastening releasing time of ornoprostil in the digestive tract than that of diclofenac or a salt thereof, or synchronizing both of them. That is, it is preferable to use a preparation prepared in such a manner that it shows a releasing pattern in which, after administration of the pharmaceutical preparation of the present invention, diclofenac or a salt thereof is released after release of ornoprostil or simultaneously therewith. In addition, it is preferable that both of ornoprostil and diclofenac or a salt thereof are released in the same region, and it is particularly preferable that they are released in the stomach. Preferred as the pharmaceutical preparation of the present invention is a combination agent which comprises ornoprostil and diclofenac or a salt thereof in one preparation.

As the combination agent of the present invention, a separation type pharmaceutical preparation can be exemplified. The separation type pharmaceutical preparation is a preparation designed in such a manner that diclofenac or a salt thereof does not directly contact with ornoprostil.

Preferable as the separation type pharmaceutical preparation of the present invention are, for example,

1) a single hard capsule preparation which comprises at least one or more of the (A), and the (D), shown below,
2) a single hard capsule preparation which comprises at least one or more of the (A) and at least one or more of the (C), shown below,
3) a single hard capsule preparation which comprises at least the (B) and at least one or more of the (C), shown below,
4) a single soft capsule preparation which comprises at least one or more of the (A), and the (D), shown below,
5) a single soft capsule preparation which comprises the (B) and at least one or more of the (C), shown below,
6) a soft capsule preparation prepared by coating the following (B) with a soft capsule coat containing diclofenac or a salt thereof,
7) a soft capsule preparation prepared by coating the following (D) with a soft capsule coat containing ornoprostil,
8) a single tablet preparation which comprises at least one or more of the (A), and the (D), shown below,
9) a single tablet preparation which comprises at least one or more of the (A) and at least one or more of the (C), shown below, and the like.

As the separation type pharmaceutical preparation, a single hard capsule preparation which contains at least one or more of (A) and (D) is preferable.

(A): A small soft capsule preparation which comprises ornoprostil and an oily solution base.

(B): A pharmaceutical composition which comprises ornoprostil and an oily solution base.

(C): A small soft capsule preparation which comprises diclofenac or a salt thereof and an oily solution base.

(D): A pharmaceutical composition which comprises diclofenac or a salt thereof

According to the present invention, the soft capsule preparation is prepared by filling a gelatin coat with, for example, liquid or suspension of an inner liquid, and it includes those which are produced by a punching method such as a rotary method or a plate method (e.g., oval type, round type, suppository type, oblong type, tube type, *etc.)* and those which are produced by a dropping method (e.g., double nozzle method, triple nozzle method, quadruple nozzle method, quintet nozzle method, *etc*.) (seamless capsules).

The gelatin coat of soft capsules uses a gelatin as the base and may be blended with a plasticizer as occasion demands.

Examples of the gelatin include an alkali-treated gelatin, an acid-treated gelatin, an enzyme-treated gelatin, a chemically modified gelatin and the like, and one or at least two of them can be used through optional formulation.

Examples of the plasticizer include concentrated glycerin, glycerin, sorbitol, maltose, glucose, maltitose, sucrose, xylitol, mannitol, erythritol, propylene glycol, polyethylene glycol and the like, and one or at least two of them can be used through optional formulation.

Kind and formulation ratio of gelatin and plasticizer can be optionally selected by taking size, shape and the like of the soft capsule of interest into consideration. In addition, a coloring agent, an antiseptic and the like cab be formulated as occasion demands.

Thickness of the coating may be such a thickness that the soft capsule preparation can keep sufficient strength, and release the active ingredient by disintegrating at a proper time when administered.

The hard capsule preparation is prepared by filling an agent into, for example, a gelatin capsule, a hydroxypropylmethylcellulose (HPMC) capsule, a pullulan capsule or a polyvinyl alcohol (PVA) capsule. As the size of hard capsules, from No. 000 to No. 5 can be exemplified, and to facilitate their taking, No. 2 to No. 5 capsules are preferable, and No. 4 capsules are more preferable.

Regarding the oily solution base of the "small soft capsule preparation which comprises ornoprostil and an oily solution base" of the above-described (A), it may be any substance which can produce the soft capsule preparation and does not spoil stability of ornoprostil. Its examples include beefsteak plant oil, rice germ oil, wheat germ oil, soybean oil, rapeseed oil, peppermint oil, sunflower oil, coconut oil, peanut oil, corn oil, safflower oil, sesame oil, cotton seed oil, palm oil, olive oil, castor oil, fish oil, cuttlefish oil, whale oil, beef tallow, lard, snake oil, propylene glycol fatty acid ester, esterificated corn oil, safflower oil fatty acid, hardened oil, middle chain fatty acid triglyceride (MCT), tricaprylin, glycerin fatty acid ester, glycerin monostearate, polysorbate 80 and the like, and one or at least two thereof may be used through an optional formulation.

Preferred as the oily solution base is middle chain fatty acid triglyceride (MCT) or tricaprylin. The middle chain fatty acid triglyceride (MCT) contains a fatty acid triglyceride having a fatty acid carbon chain length of from 4 to 10 as the main component. The fatty acid moiety of glyceride consists of caproic acid, caprylic acid, capric acid, lauric acid and the like, and all of the fatty acid moieties may be the same or different from one another. Examples include tricaproin, tricaprylin, tricaprin, trilaurin and the like. Preferred are tricaprylin and tricaprin, and more preferred is tricaprylin. In addition, one or at least two species of high viscosity additive agent may be optionally formulated as occasion demands. The high viscosity additive agent may be any substance which can be used for preventing liquid leaking and uniformly filing capsules, and its examples include waxes (e.g., castor wax, bleached beeswax, beeswax, carnauba wax, rice bran oil, *etc*.), cacao butter, hardened oil, soybean oil unsaponifiable matter, soybean hardened oil and the like.

Size, shape and numbers of the small soft capsule preparation of (A) can be optionally selected by taking into consideration the size, shape and the like of hard capsules, soft capsules, tablets and the like containing the small soft capsule preparation of (A). The size (outer diameter) of the small soft capsule preparation is preferably from about 0.5 mm to about 8 mm, more preferably from about 1 mm to about 7 mm, more preferably from about 2 mm to about 6 mm, further preferably from about 2 mm to about 4 mm.

In order to further improve dispersibility and fast-acting property of (A) in the stomach when this preparation is administered, it is preferable to increase the number of the small soft capsule preparation. A seamless capsule is preferable as the small soft capsule, and outer diameter of the seamless capsule is preferably from about 0.1 mm to 4 mm, more preferably from about 0.1 mm to 2 mm. As the number of the small soft capsule preparation, for example, from 1 to 400 can be cited. As the number of the small soft capsule preparation when outer diameter of the small soft capsule preparation is small, it is preferably from 10 to 400, more preferably from 20 to 100, further preferably from 30 to 50. Also, as the number of the small soft capsule preparation when outer diameter of the small soft capsule preparation is relatively large, it is preferably from 1 to 20, more preferably from 1 to 10, further preferably 1 or 2. When the small soft capsule preparation of (A) is a seamless capsule preparation, the "%" by weight of the small soft capsule preparation coating based on 100% by weight is preferably from about 5 to 60%, more preferably from about 10 to 50%, further preferably from 20 to 45%. The "%" by weight of the oily solution base based on 100% by weight of the small soft capsule preparation of (A) is preferably from 40 to 95%, more preferably from 50 to 90%, further preferably from 55 to 80%.

The "pharmaceutical composition which comprises ornoprostil and an oily solution base" of the above-described (B) includes, for example, a solution or suspension of ornoprostil in an oily solution base. As the oily solution base, it may be any substance which does not spoil stability of ornoprostil. Its examples include beefsteak plant oil, rice germ oil, wheat germ oil, soybean oil, rapeseed oil, peppermint oil, sunflower oil, coconut oil, peanut oil, corn oil, safflower oil, sesame oil, cotton seed oil, palm oil, olive oil, castor oil, fish oil, cuttlefish oil, whale oil, beef tallow, lard, snake oil, propylene glycol fatty acid ester, esterificated corn oil, safflower oil fatty acid, hardened oil, middle chain fatty acid triglyceride (MCT), tricaprylin, glycerin fatty acid ester, glycerin monostearate, polysorbate 80 and the like, and one or at least two thereof may be used through an optional formulation.

Preferred as the oily solution base is middle chain fatty acid triglyceride (MCT) or tricaprylin. The middle chain fatty acid triglyceride (MCT) contains a fatty acid triglyceride having a fatty acid carbon chain length of from 4 to 10 as the main component. The fatty acid moiety of glyceride consists of caproic acid, caprylic acid, capric acid, lauric acid and the like, and all of the fatty acid moieties may be the same or different from one another. Examples include tricaproin, tricaprylin, tricaprin, trilaurin and the like. Preferred are tricaprylin and tricaprin, and more preferred is tricaprylin. In addition, one or at least two species of high viscosity additive agent may be optionally formulated as occasion demands. The high viscosity additive agent may be any substance which can be used for preventing liquid leaking and uniformly filing capsules, and its examples include waxes (e.g., castor wax, bleached beeswax, beeswax, carnauba wax, rice bran oil, *etc.*), cacao butter, hardened oil, soybean oil unsaponifiable matter, soybean hardened oil and the like.

As the oily solution base of the "small soft capsule preparation which comprises diclofenac or a salt thereof and an oily solution base" of the above-described (C), it may be any substance which can produce the soft capsule preparation and does not spoil stability of diclofenac. Examples include beefsteak plant oil, rice germ oil, wheat germ oil, soybean oil, rapeseed oil, peppermint oil, sunflower oil, coconut oil, peanut oil, corn oil, safflower oil, sesame oil, cotton seed oil, palm oil, olive oil, castor oil, fish oil, cuttlefish oil, whale oil, beef tallow, lard, snake oil, propylene glycol fatty acid ester, esterificated corn oil, safflower oil fatty acid, hardened oil, middle chain fatty acid triglyceride (MCT), tricaprylin, glycerin fatty acid ester, glycerin monostearate, polysorbate 80, polyethylene glycol and the like, and one or at least two thereof may be used through an optional formulation.

Preferred as the oily solution base is middle chain fatty acid triglyceride (MCT) or tricaprylin. The middle chain fatty acid triglyceride (MCT) contains a fatty acid triglyceride having a fatty acid carbon chain length of from 4 to 10 as the main component. The fatty acid moiety of glyceride consists of caproic acid, caprylic acid, capric acid, lauric acid and the like, and all of the fatty acid moieties may be the same or different from one another. Examples include tricaproin, tricaprylin, tricaprin, trilaurin and the like. In addition, one or at least two species of high viscosity additive agent may be optionally formulated as occasion demands. The high viscosity additive agent may be any substance which can be used for preventing liquid leaking and uniformly filing capsules, and its examples include waxes (e.g., castor wax, bleached beeswax, beeswax, carnauba wax, rice bran oil, *etc.*), cacao butter, hardened oil, soybean oil unsaponifiable matter, soybean hardened oil and the like. Size, shape and numbers of the small soft capsule preparation of (C) can be optionally selected by taking into consideration the size, shape and the like of hard capsules, soft capsules, tablets and the like containing the small soft capsule preparation of (C). The size (outer diameter) of the small soft capsule preparation is preferably from about 0.5 mm to about 8 mm, more preferably from about 1 mm to about 7 mm, more preferably from about 2 mm to about 6 mm, further preferably from about 2 mm to about 4 mm. As the number of the small soft capsule preparation, for example, from 1 to 400 can be cited. As the number of the small soft capsule preparation when outer diameter of the small soft capsule preparation is small, it is preferably from 10 to 400, more preferably from 20 to 100, further preferably from 30 to 50. Also, as the number of the small soft capsule preparation when outer diameter of the small soft capsule preparation is relatively large, it is preferably from 1 to 20, more preferably from 1 to 10, further preferably 1 or 2. The "%" by weight of the small soft capsule preparation coating based on 100% by weight of the small soft capsule preparation of (C) is preferably from about 20 to 70%, more preferably from about 30 to 60%, further preferably from about 40 to 50%. The "%" by weight of the oily solution base based on 100% by weight of the small soft capsule preparation of (C) is preferably from 30 to 80%, more preferably from 40 to 70%, further preferably from 50 to 60%.

The "pharmaceutical composition which comprises diclofenac or a salt thereof" of the above-described (D) includes a pharmaceutical composition comprising diclofenac or a salt thereof and an additive agent, (D-2) a pharmaceutical composition comprising diclofenac or a salt thereof and an oily solution base, and the like. In addition, the pharmaceutical composition comprising diclofenac or a salt thereof and an additive agent may have a form of powders, fine subtilaes or granules, of which granules are preferable. As the oily solution base in the pharmaceutical composition comprising diclofenac or a salt thereof and an oily solution base of (D-2), it may be any substance which does not spoil stability of diclofenac or a salt thereof, and its examples include beefsteak plant oil, rice germ oil, wheat germ oil, soybean oil, rapeseed oil, peppermint oil, sunflower oil, coconut oil, peanut oil, corn oil, safflower oil, sesame oil, cotton seed oil, palm oil, olive oil, castor oil, fish oil, cuttlefish oil, whale oil, beef tallow, lard, snake oil, propylene glycol fatty acid ester, esterificated corn oil, safflower oil fatty acid, hardened oil, middle chain fatty acid triglyceride (MCT), tricaprylin, glycerin fatty acid ester, glycerin monostearate, polysorbate 80, polyethylene glycol and the like, and one or at least two thereof may be used through an optional formulation.

Preferred as the oily solution base is middle chain fatty acid triglyceride (MCT) or tricaprylin. The middle chain fatty acid triglyceride (MCT) contains a fatty acid triglyceride having a fatty acid carbon chain length of from 4 to 10 as the main component. The fatty acid moiety of glyceride consists of caproic acid, caprylic acid, capric acid, lauric acid and the like, and all of the fatty acid moieties may be the same or different from one another. Examples include tricaproin, tricaprylin, tricaprin, trilaurin and the like.

In addition, one or at least two species of high viscosity additive agent may be optionally formulated as occasion demands. The high viscosity additive agent may be any substance which can be used for preventing liquid leaking and uniformly filing capsules, and its examples include waxes (e.g., castor wax, bleached beeswax, beeswax, carnauba wax, rice bran oil, *etc.*), cacao butter, hardened oil, soybean oil unsaponifiable matter, soybean hardened oil and the like. Examples of the additive agent in the pharmaceutical composition comprising diclofenac or a salt thereof and an additive agent include those in which one or at least two of excipients, binders, disintegrators, correctives, surfactants, aromatics, lubricants, coloring agents, antioxidants, masking agents, anti-electrostatic agents, superplasticizers, moistening agents and the like are optionally blended.

Examples of the excipients include saccharides (e.g., glucose, fructose, maltose, milk sugar (lactose), isomerized lactose (lactulose), reduced lactose (lactitol), sucrose, D-mannitol, erythritol, maltitol, xylitol, palatinose, *etc.*), trehalose, sorbitol, corn starch, potato starch, wheat starch, rice starch, crystalline cellulose (e.g., Avicel, Ceolas, *etc.*), silicic anhydride, anhydrous calcium phosphate, precipitated calcium carbonate, calcium silicate and the like.

Examples of the binders include hydroxypropylcellulose, hydroxypropylmethylcellulose, povidone, polyvinyl pyrrolidone (e.g., Colidon 25), methyl cellulose, polyvinyl alcohol, carboxymethylcellulose sodium, partial α-starch, α-starch, sodium alginate, pullulan, powdered acacia, gelatin and the like.

Examples of the disintegrators include low substitution degree hydroxypropylcellulose, carmellose, carmellose calcium, carboxymethylstarch sodium, crosscarmellose sodium, crosspovidone, hydroxypropylstarch, corn starch and the like.

Examples of the correctives include sucrose, D-sorbitol, xylitol, citric acid, ascorbic acid, tartaric acid, malic acid, aspartame, acesulfame potassium, thaumatin, saccharin sodium, dipotassium glycyrrhetinate, sodium glutamate, sodium 5'-inosinate, sodium 5'-guanylate and the like.

Examples of the surfactants include polysorbate (Polysorbate 80, *etc.*), polyoxyethylene-polyoxypropylene copolymer, sodium lauryl sulfate and the like.

Examples of the aromatics include lemon oil, orange oil, menthol, peppermint oil and the like.

Examples of the lubricants include magnesium stearate, calcium stearate, sucrose fatty acid ester, stearyl fumarate sodium, stearic acid, talc, polyethylene glycol and the like.

Examples of the coloring agents include titanium oxide, Food Yellow No. 5, Food Blue No. 2, iron sesquioxide, yellow iron sesquioxide and the like.

Examples of the antioxidants include sodium ascorbate, L-cysteine, sodium sulfite, vitamin E and the like.

Examples of the masking agents include titanium oxide and the like.

Examples of the anti-electrostatic agents include talc, titanium oxide and the like.

Examples of the superplasticizers include soft silicic anhydride, talc, hydrous silicon dioxide and the like.

Examples of the moistening agents include polysorbate 80, sodium lauryl sulfate, sucrose fatty acid ester, macrogol, hydroxypropylcellulose (HPC) and the like.

Preferred as the additive agent are those in which one or at least two of excipients and binders are optionally formulated, and preferred as the excipients are saccharides, corn starch, crystalline cellulose or anhydrous calcium phosphate, wherein preferred as the saccharides is lactose, D-mannitol, erythritol or xylitol. Also, preferred as the binders are hydroxypropylcellulose, polyvinyl pyrrolidone or polyvinyl alcohol.

Preferred as a pharmaceutical composition comprising diclofenac or a salt thereof and an additive agent is the (D-1) pharmaceutical composition comprising diclofenac or a salt thereof and an excipient.

The pharmaceutical composition comprising diclofenac or a salt thereof and an additive agent may have a form of powders, fine subtilaes or granules, of which granules are preferable.

For miniaturization and dissolution property of the pharmaceutical preparation of the present invention, the average particle size of the granules is preferably from 50 µm to 1,500 µm, more preferably from 200 µm to 600 µm. Bulk density of granules of the pharmaceutical composition of (D-1) is preferably from about 0.4 g/ml to 0.9 g/ml, more preferably from 0.5 g/ml to 0.8 g/ml. The "%" by weight of the excipient based on 100% by weight of the pharmaceutical composition of (D-1) is preferably from 20 to 95%, more preferably from 30 to 90%, further preferably from 50 to 85%.

The "small soft capsule preparation which comprises ornoprostil and an oily solution base" of (A) and the "small soft capsule preparation which comprises diclofenac or a salt thereof and an oily solution base" of (C) can be produced in accordance with a conventionally known method, and it can be carried out, for example, by dissolving or

EP 1 547 587 A1

suspending the active ingredient in an oily solution base, further adding a high viscosity additive agent as occasion demands, treating this using a continuous soft capsule producing machine such as a punching method (e.g., rotary method, plate method, *etc*.) or a dropping method (e.g., double nozzle method, triple nozzle method, quadruple nozzle method, quintet nozzle method, *etc*.), and then filling a gelatin coat with the thus obtained treated liquid. Powders, fine subtilaes and granules of the "pharmaceutical preparation composition comprising diclofenac or a salt thereof and saccharides" can be produced by a conventionally known granulation method, or can be produced without granulation by simply mixing with an additive agent. Examples of the granulation method include stirring granulation, fluidized bed granulation, dry granulation, roller stirring fluidized bed granulation and the like.

A size of from No. 2 to No. 5 is preferable as the hard capsule in the "single hard capsule preparation which comprises at least one or more of (A) and (D)" and more preferable is No. 3 or No. 4 capsule, and a seamless capsule preparation is preferable as the soft capsule preparation of (A), wherein outer diameter of the seamless capsule preparation is preferably from about 0.1 mm to 6 mm. The "%" by weight of the seamless capsule coating based on 100% by weight of seamless capsule preparation is preferably from about 5 to 60%, more preferably from about 10 to 50%, further preferably from 20 to 45%, and the "%" by weight of the oily solution base based on 100% by weight of seamless capsule preparation is preferably from 40 to 95%, more preferably from 50 to 90%, further preferably from 55 to 80%. The pharmaceutical preparation composition of (D) comprising diclofenac or a salt thereof is preferably the (D-1) pharmaceutical preparation composition comprising diclofenac or a salt thereof and an excipient. Preferred as the excipient is a saccharide, corn starch, crystalline cellulose or anhydrous calcium phosphate, wherein preferred as the saccharide is lactose, D-mannitol, erythritol or xylitol. The pharmaceutical preparation composition of (D-1) is preferably a granule preparation, and average particle size of the granule preparation is preferably from 50 μm to 1,500 μm, more preferably from 200 μm to 600 μm. Bulk density of granule preparation of the pharmaceutical preparation composition of (D-1) is preferably from about 0.4 g/ml to 0.9 g/ml, more preferably from 0.5 g/ml to 0.8 g/ml. The "%" by weight of the excipient based on 100% by weight of the pharmaceutical preparation composition of (D-1) is preferably from 20 to 95%, more preferably from 30 to 90%, further preferably from 50 to 85%. In addition, when the size of hard capsule is No. 0, the "%" by weight of the excipient based on 100% by weight of the pharmaceutical preparation composition of (D-1) is preferably from 70 to 95%, more preferably from 80 to 95%. When the size of hard capsule is No. 1, the "%" by weight of the excipient based on 100% by weight of the pharmaceutical preparation composition of (D-1) is preferably from 50 to 95%, more preferably from 80 to 95%. When the size of hard capsule is No. 2, the "%" by weight of the excipient based on 100% by weight of the pharmaceutical preparation composition of (D-1) is preferably from 40 to 95%, more preferably from 75 to 95%. When the size of hard capsule is No. 3, the "%" by weight of the excipient based on 100% by weight of the pharmaceutical preparation composition of (D-1) is preferably from 20 to 90%, more preferably from 70 to 90%. When the size of hard capsule is No. 4, the "%" by weight of the excipient based on 100% by weight of the pharmaceutical preparation composition of (D-1) is preferably from 20 to 90%, more preferably from 50 to 85%. When the size of hard capsule is No. 5, the "%" by weight of the excipient based on 100% by weight of the pharmaceutical preparation composition of (D-1) is preferably from 20 to 80%, more preferably from 50 to 75%.

The "single hard capsule preparation which comprises at least one or more of (A) and (D)" can be produced in accordance with a conventionally known method, and the hard capsule preparation of interest is obtained by filling a hard capsule with the components (A) and (D) and, as occasion demands, an additive agent, using a capsule filling machine, and further sealing the capsule connecting part as occasion demands.

A size of from No. 2 to No. 5 is preferable for the hard capsule in the "single hard capsule preparation which comprises at least one or more of (A) and (C)", and more preferable is a No. 4 capsule. The "single hard capsule preparation which comprises at least one or more of (A) and (C)" can be produced in accordance with a conventionally known method, and the hard capsule preparation of interest is obtained by filling a hard capsule with the components (A) and (C) and, as occasion demands, an additive agent, using a capsule filling machine, and further sealing the capsule connecting part as occasion demands.

A size of from No. 2 to No. 5 is preferable for the hard capsule in the "single hard capsule preparation which comprises (B) and at least one or more of (C)", and more preferable is a No. 4 capsule. The "hard capsule preparation which comprises components (B) and (C)" can be produced in accordance with a conventionally known method, and the hard capsule preparation of interest is obtained by filling a hard capsule with the components (B) and (C) and, as occasion demands, an additive agent, using a capsule filling machine, and further sealing the capsule connecting part as occasion demands.

The "single soft capsule preparation which comprises at least one or more of (A) and (D)" can be produced in accordance with a conventionally known method, and it can be carried out by using a continuous soft capsule producing apparatus of, for example, a punching method (e.g., rotary method, plate method, *etc*.) or a dropping method (e.g., double nozzle method, triple nozzle method, quadruple nozzle method, quintet nozzle method, *etc*.).

The "single soft capsule preparation which comprises (B) and at least one or more of (C)" can be produced in accordance with a conventionally known method, and it can be carried out by using a continuous soft capsule producing apparatus of, for example, a punching method (e.g., rotary method, plate method, *etc*.) or a dropping method (e.g., double nozzle method, triple nozzle method, quadruple nozzle method, quintet nozzle method, *etc.*).

As the "soft capsule preparation prepared by coating (B) with a soft capsule coat containing diclofenac or a salt thereof", a mixture of a gelatin coat and diclofenac or a salt thereof can be exemplified as the soft capsule coat containing diclofenac or a salt thereof The gelatin coat may be of any origin, with the proviso that it does not spoil stability of diclofenac or a salt thereof, and its examples include an alkali-treated gelatin, an acid-treated gelatin, an enzyme-treated gelatin, a chemically modified gelatin and the like, and one or at least two of them can be used through optional formulation, wherein one or at least two of plasticizers may be optionally formulated.

Examples of the plasticizer include concentrated glycerin, glycerin, sorbitol, maltose, glucose, maltitose, sucrose, xylitol, mannitol, erythritol, propylene glycol, polyethylene glycol and the like.

Kind and formulation ratio of gelatin and plasticizer can be optionally selected by taking size, shape and the like of the soft capsule of interest into consideration. In addition, a coloring agent, an antiseptic and the like can be formulated as occasion demands. Thickness of the coating may be such a thickness that the soft capsule preparation can keep sufficient strength and release the active ingredient by disintegrating at a proper time when administered, and it may have a multi-layer of two layers or more for the purpose of further improving separation ability between diclofenac or a salt thereof and ornoprostil. The "%" by weight of diclofenac or a salt thereof based on the coat mass is preferably from about 1 to 20%, more preferably from 5 to 15%, further preferably from 8 to 12%. When the coat is made into a multi-layer, taking feeling of this preparation and stability of ornoprostil and diclofenac or a salt thereof are taken into consideration, so that, in a coat layer other than the coat layer mainly containing diclofenac or a salt thereof for example, the "%" by weight of diclofenac or a salt thereof based on the coat layer mass is preferably from 0 to 5%, more preferably from 0 to 1%.

The soft capsule preparation prepared by coating (B) with a soft capsule coat containing diclofenac or a salt thereof can be produced in accordance with a conventionally known method, and it can be carried out by filling the above-described coating with (B) using a continuous soft capsule producing apparatus of, for example, a punching method (e.g., rotary method, plate method, *etc.*) or a dropping method (e.g., double nozzle method, triple nozzle method, quadruple nozzle method, quintet nozzle method, *etc.*).

Regarding the "soft capsule preparation prepared by coating (D) with a soft capsule coat containing ornoprostil", a mixture of a gelatin coat and ornoprostil can be exemplified as the soft capsule coat containing ornoprostil. The gelatin coat may be of any origin, with the proviso that it does not spoil stability of ornoprostil, and its examples include an alkali-treated gelatin, an acid-treated gelatin, an enzyme-treated gelatin, a chemically modified gelatin and the like, and one or at least two of them can be used through optional formulation, wherein one or at least two of plasticizers may be optionally formulated.

Examples of the plasticizer include concentrated glycerin, glycerin, sorbitol, maltose, glucose, maltitose, sucrose, xylitol, mannitol, erythritol, propylene glycol, polyethylene glycol and the like.

Kind and formulation ratio of gelatin and plasticizer can be optionally selected by taking size, shape and the like of the soft capsule of interest into consideration. In addition, a coloring agent, an antiseptic and the like cab be formulated as occasion demands. Thickness of the coating may be such a thickness that the soft capsule preparation can keep sufficient strength and release the active ingredient by disintegrating at a proper time when administered, and it may have a multi-layer of two layers or more for the purpose of further improving separation ability between diclofenac or a salt thereof and ornoprostil.

A solution or dispersion of diclofenac or a salt thereof diclofenac or a salt thereof in an oily solution base is preferable as the (D). The soft capsule preparation prepared by coating (D) with a soft capsule coat containing ornoprostil can be produced in accordance with a conventionally known method, and it can be carried out by filling the above-described coating with (D) using a continuous soft capsule producing apparatus of, for example, a punching method (e.g., rotary method, plate method, *etc.*) or a dropping method (e.g., double nozzle method, triple nozzle method, quadruple nozzle method, quintet nozzle method, *etc.*).

The "single tablet preparation which comprises at least one or more of (A) and (D)" can be produced in accordance with a conventionally known method, and it can be carried out, for example, by making (A), (D) and one or at least two additive agents (have the same meaning as the additive agents in the above-described pharmaceutical preparation composition comprising diclofenac or a salt thereof and an additive agent) into a tablet by, for example, a direct powder compression method, a wet granule compression method, a dry granule compression method or the like.

The "single tablet preparation which comprises at least one or more of (A) and at least one or more of (C)" can be produced in accordance with a conventionally known method, and it can be carried out, for example, by making (A), (C) and one or at least two additive agents (have the same meaning as the additive agents in the above-described pharmaceutical preparation composition comprising diclofenac or a salt thereof and an additive agent) into a tablet by, for example, a direct powder compression method, a wet granule compression method, a dry granule compression method or the like.

Both of diclofenac or a salt thereof and ornoprostil as the active ingredients of the pharmaceutical composition of the present invention are agents which are already on the market and can be produced by conventionally known methods.

An effective amount (dose) of diclofenac or a salt thereof varies depending on the age, body weight, symptoms, therapeutic effect, administration method, treating time and the like, but from 10 mg to 500 mg, preferably from 10 mg to 200 mg, more preferably from 25 mg to 150 mg can be exemplified as a daily dose per adult. The pharmaceutical preparation containing diclofenac or a salt thereof may be administered once a day or by dividing it into several times (e.g., 2 to 4 times, preferably 3 or 4 times). As a preferable administration method, it is oral administration.

An effective amount (dose) of ornoprostil varies depending on the age, body weight, symptoms, therapeutic effect, administration method, treating time and the like, but side effects of diclofenac or a salt thereof can be reduced when a biological catalytic amount of ornoprostil is administered based on diclofenac or a salt thereof. The biological catalytic amount includes an amount of from 0.1 μg to 200 μg, preferably from 1 μg to 100 μg, more preferably from 2.5 μg to 80 μg, and most preferably from 5 μg to 30 μg. The pharmaceutical preparation containing ornoprostil may be administered once a day or by dividing it into several times (e.g., 2 to 4 times, preferably 3 or 4 times). As a preferable administration method, it is oral administration.

As a matter of course, since doses of diclofenac or a salt thereof and ornoprostil change depending on various conditions, there is a case in which smaller amounts than the above-described doses are sufficient or a case in which amounts exceeding the ranges are necessary.

According to the combination agent of the present invention (separation type pharmaceutical preparation), combination of the amounts of diclofenac or a salt thereof and ornoprostil in one pharmaceutical preparation is preferably from 2.5 μg to 80 μg of ornoprostil based on 10 mg to 200 mg of diclofenac or a salt thereof, more preferably from 5 μg to 30 μg of ornoprostil based on 25 mg to 150 mg of diclofenac or a salt thereof, further preferably 5 μg of ornoprostil based on 25 mg of diclofenac or a salt thereof. The combination agent of the present invention (separation type pharmaceutical preparation) may be administered once a day or dividing it into several times, but it is preferable to administer preferably 3 or 4 times a day. The administration timing is not limited, but is preferably after meals.

The ratio of weight (biological catalytic amount) of ornoprostil to one weight part of diclofenac or a salt thereof is preferably from 1/100,000 to 1/1,000, more preferably from 1/50,000 to 1/2,500, further preferably from 1/25,000 to 1/5,000.

PG which protects gastrointestinal mucous membrane disorders from endogenous factors, such as gastric acid, pepsin, and bile acid, and exogenous factors, such as *H. pylori* and ethanol, includes PGI2 (IP receptor) and PGE2 (EP1, EP2, EP3 and EP4 receptors) mainly biosynthesized by COX-1, and their activity is called site protection activity. PG derivatives which inhibit NSAID-induced digestive disorders have different affinity for these PG receptors and different activity characteristics. For example, among the PG receptors, an ornoprostil active body has markedly strong affinity for IP receptors and also has strong affinity for EP1, EP3 and EP4 receptors. On the other hand, a misoprostol active body has strong affinity for EP3 and EP4 receptors but has weak affinity for EP1 receptor, and hardly has affinity for IP receptor. Accordingly, misoprostol has strong acid secretion inhibitory activity but its site protection activity is weak. On the other hand, ornoprostil has weak acid secretion inhibitory activity but its site protection activity is strong.

According to the separation type pharmaceutical preparation of the present invention which can improve stability of PG derivatives, other NSAID (for example, acemetacin, aspirin, loxoprofen, indometacin, ibuprofen, selective COX-2 inhibitors (e.g., selecoxib, rofecoxib, meloxicam, *etc.*) or the like) can be used instead of diclofenac or a salt thereof in response to respective purposes, and other PG derivative (e.g., misoprostol, enprostil, *etc.*) can be used instead of ornoprostil.

Industrial Applicability

Application to pharmaceutical preparations:

The agent for reducing side effects of the present invention has antipyretic, analgesic and antiphlogistic activities and the like, and can be used as a therapeutic and/or preventive agent for, for example, articular rheumatism, rheumatic fever, osteoarthritis, tetanic spondylitis, spondylosis deformans, periarthritis (e.g., scapulohumeral periarthritis, *etc.*), cervico-omo-brachial syndrome, tendon marrow inflammation, neuralgia, joint pain, muscle pain, low back pain, pain or inflammation after operation, injury or tooth extraction, sprain pain, contusion pain, toothache, headache, pain of gout, menstrual pain, dysmenorrhea, after labor pain, pelvic inflammation, tergum inflammation, cystitis, anterior chamber inflammation and/or acute upper respiratory inflammation (e.g., common cold, cold syndrome, pharyngitis, laryngitis, *etc.)* and the like.

In addition, the agent for reducing side effects of the present invention (combination agent) can reduce side effects of diclofenac or a salt thereof (e.g., digestive disorders such as gastric mucosal disorder, gastric ulcer, duodenal ulcer and stomach region discomfort, worsening of nephropathy, *etc.*).

Effects of the present invention:

The agent for reducing side effects of the present invention reduces side effects of diclofenac or a salt thereof (e.g., digestive disorders, nephropathy, *etc*.), and side effects such as diarrhea and vomiting are also considerably less. In addition, since the combination agent of the present invention is a separation type pharmaceutical preparation, stability of ornoprostil can be improved.

Best Mode for Carrying Out the Invention

Preparation Examples and Test Examples (pharmacological test, dissolution test of preparations, blood dynamic test, stability test) are shown in the following as Examples, but these are for thoroughly understanding the present invention and do not limit the scope of the present invention.
Abbreviations used in respective examples represent the following meanings.

Di = diclofenac sodium, In = indometacin, Ac = acemetacin, OU =

ornoprostil, MI = misoprostol, GC = glycerol.

Preparation Example 1: Soft capsules-1

To 528 g of tricaprylin, 72 g of glycerin monostearate (MGS-B) was added, and MGS-B was dissolved by heating this to about 65°C. After cooling, 300 g of ornoprostil-containing tricaprylin solution (60 mg/300 g) was added thereto and mixed. Subsequently, 300 g of diclofenac sodium was added thereto and mixed, and undissolved lumps of flour were pulverized using Mycolloider and filtered through a 180 μm (80 mesh) screen. The filtered liquid was degassed under a reduced pressure to obtain a formula liquid.
The above-described formula liquid was made into soft capsules by carrying out filling, shaping and drying in the usual method using a soft capsule coat containing gelatin, concentrated glycerin and D-sorbitol solution, thereby producing soft capsules (a total of 7,200 capsules) of the following formula.

| Formula of soft capsules-1 | |
| --- | --- |
| Components of formula liquid | Amount (mg) |
| Ornoprostil | 0.005 |
| Diclofenac sodium | 25 |
| Glycerin monostearate | 6 |
| Tricaprylin | 69 |
| Components of soft capsule coating | |
| Gelatin | 50 |
| Concentrated glycerin | 5 |
| D-sorbitol solution | 15 |
| Total | 170 |

Preparation Example 2: Soft capsules-2

To 600 g of tricaprylin, 300 g of ornoprostil-containing tricaprylin solution (60 mg/300 g) was added, followed by mixing. Subsequently, 300 g of diclofenac sodium was added thereto and mixed, and undissolved lumps of flour were pulverized using Mycolloider and filtered through a 180 μm (80 mesh) screen. The filtered liquid was degassed under a reduced pressure to obtain a formula liquid.
The above-described formula liquid was made into soft capsules by carrying out filling, shaping and drying in the usual method using a soft capsule coat containing gelatin, concentrated glycerin and D-sorbitol solution, thereby producing soft capsules (a total of 7,200 capsules) of the following formula.

| Formula of soft capsules-2 | |
|---|---|
| Components of formula liquid | Amount (mg) |
| Ornoprostil | 0.005 |
| Diclofenac sodium | 25 |
| Tricaprylin | 75 |
| Components of soft capsule coating | |
| Gelatin | 50 |
| Concentrated glycerin | 5 |
| D-sorbitol solution | 15 |
| Total | 170 |

Preparation Example 3: Gelatin hard capsules

To 540 g of tricaprylin, 10 g of ornoprostil-containing tricaprylin solution (200 mg/200 g) was added, followed by mixing. Subsequently, 50 g of diclofenac sodium was added thereto and mixed and then filtered through a 150 μm (100 mesh) screen.

Hard capsules (a total of 1,200 capsules) of the following formula were produced by filling No. 2 gelatin hard capsules with the above-described formula liquid in the usual method, followed by sealing and drying.

| Formula of gelatin hard capsules | |
|---|---|
| Components of formula liquid | Amount (mg) |
| Ornoprostil | 0.005 |
| Diclofenac sodium | 25 |
| Tricaprylin | 275 |
| Total | 300 |

Preparation Example 4: HPMC hard capsules-1

HPMC hard capsules (a total of 1,200 capsules) of the following formula were produced by carrying out the same operation of Preparation Example 3, using No. 2 HPMC hard capsules instead of the No. 2 gelatin hard capsules.

| Formula of HPMC hard capsules-1 | |
|---|---|
| Components of formula liquid | Amount (mg) |
| Ornoprostil | 0.005 |
| Diclofenac sodium | 25 |
| Tricaprylin | 275 |
| Total | 300 |

Preparation Example 5: HPMC hard capsules-2

To 523.5 g of tricaprylin, 16.5 g of hardened oil was added, and the hardened oil was dissolved under heating up to about 90°C. After cooling, 10 g of ornoprostil-containing tricaprylin solution (200 mg/200 g) was added thereto and mixed. Subsequently, 50 g of dielofenac sodium was added thereto, mixed using a mixer and then filtered through a 150 μm (100 mesh) screen.

HPMC hard capsules (a total of 1,200 capsules) of the following formula were produced by filling No. 2 HPMC hard capsules with the above-described formula liquid in the usual method, followed by sealing and drying.

| Formula of HPMC hard capsules-2 | |
|---|---|
| Components of formula liquid | Amount (mg) |
| Ornoprostil | 0.005 |
| Diclofenac sodium | 25 |
| Hardened oil | 8.25 |
| Tricaprylin | 266.75 |
| Total | 300 |

Preparation Example 6: HPMC hard capsules-3

HPMC hard capsules (a total of 1,200 capsules) of the following formula were produced by carrying out the same operation of Preparation Example 3, using No. 2 HPMC hard capsules instead of the No. 2 gelatin hard capsules.

| Formula of HPMC hard capsules-3 | |
|---|---|
| Components of formula liquid | Amount (mg) |
| Ornoprostil | 0.005 |
| Diclofenac sodium | 50 |
| Tricaprylin | 250 |
| Total | 300 |

Preparation Example 7: HPMC hard capsules-4

HPMC hard capsules (a total of 1,200 capsules) of the following formula were produced by carrying out the same operation of Preparation Example 3, using No. 2 HPMC hard capsules instead of the No. 2 gelatin hard capsules.

| Formula of HPMC hard capsules-4 | |
|---|---|
| Components of formula liquid | Amount (mg) |
| Ornoprostil | 0.005 |
| Diclofenac sodium | 25 |
| Tricaprylin | 255 |
| Total | 280.005 |

Preparation Example 8: Separation type pharmaceutical preparation-1

1) Production of ornoprostil-containing seamless capsules (Composition 1)

In 83.3 g of tricaprylin, 50 mg of ornoprostil was dissolved, and seamless capsules (a total of 5,000 capsules) having an outer diameter of 3 mm were prepared by a conventional method using a coating containing 54 g of gelatin and 6 g of concentrated glycerin.

2) Production of diclofenac sodium granules (Composition 2)

According to the usual method, 25 g of diclofenac sodium, 44 g of lactose, 22 g of corn starch, 45 g of Avicel and 25.4 g of Colidon were granulated, dried and screened to obtain granules having an average particle size of 420 $\mu$m.

3) Production of gelatin hard capsules

Gelatin hard capsules (a total of 500 capsules) of the following formula were produced by filling No. 4 gelatin hard capsule with one of the above-described seamless capsules (Composition 1) and 140 mg of the granules (Composition 2).

| Formula of separation type pharmaceutical preparation-1 (gelatin hard capsules) | |
| --- | --- |
| Seamless capsule | Amount (mg) |
| Components of formula liquid | |
| Ornoprostil | 0.005 |
| Tricaprylin | 8.33 |
| Components of capsule coating | |
| Gelatin | 5 |
| Concentrated glycerin | 0.56 |
| Formula of granules | |
| Diclofenac sodium | 25 |
| Lactose | 44 |
| Corn starch | 22 |
| Avicel | 45 |
| Colidon 25 | 4 |
| Total | 153.895 |

Preparation Example 9: Separation type preparation-2

1) Production of diclofenac sodium-containing soft capsules (Composition 1)

To 900 g of middle chain fatty acid triglyceride (MCT), 300 g of diclofenac sodium was added, followed by stirring, and undissolved lumps of flour were pulverized using Mycolloider and filtered through a 180 µm (80 mesh) screen. The filtered liquid was degassed under a reduced pressure to obtain a formula liquid.

The above-described formula liquid was made into soft capsules by carrying out filling, shaping and drying in the usual method using a soft capsule coat containing gelatin and concentrated glycerin, thereby producing round type soft capsules (a total of 14,400 capsules) having an outer diameter of 5.5 mm and containing 12.5 mg of diclofenac sodium per 1 capsule.

2) Production of ornoprostil-containing liquid (Composition 2)

To 500 g of tricaprylin, 50 mg of ornoprostil was added, followed by mixing to obtain the ornoprostil-containing liquid.

3) Production of HPMC hard capsules

HPMC hard capsules (a total of 2,000 capsules) of the following formula were produced by filling No. 4 HPMC hard capsule with two of the above-described soft capsules (Composition 1) and 50 mg of the ornoprostil-containing liquid (Composition 2), followed by sealing and drying.

| Formula of separation type pharmaceutical preparation-2 (HPMC hard capsules) | |
|---|---|
| Diclofenac-containing soft capsules | Amount (mg) |
| Components of formula liquid | |
| Diclofenac sodium | 12.5×2 |
| MCT | 37.5×2 |
| Components of capsule coating | |
| Gelatin | 35.4×2 |
| Concentrated glycerin | 8.15×2 |
| Formula of ornoprostil-containing liquid | |
| Ornoprostil | 0.005 |
| Tricaprylin | 50 |
| Total | 237.105 |

Preparation Example 10: Separation type preparation-3

1) Production of ornoprostil-containing soft capsules

In 500 g of tricaprylin, 50 mg of ornoprostil was dissolved, followed by mixing to obtain a formula liquid. The above-described formula liquid was made into soft capsules by carrying out filling, shaping and drying in the usual method using a soft capsule coat containing gelatin and concentrated glycerin, thereby producing round type soft capsules (a total of 8,000 capsules) having an outer diameter of 5.5 mm and containing 5 µg of ornoprostil per 1 capsule.
A No. 2 gelatin hard capsule was filled with one of the above-described soft capsules and two of the diclofenac sodium-containing soft capsules produced in Preparation Example 9 in the usual method, thereby producing gelatin hard capsules (a total of 2,000 capsules) of the following formula.

| Formula of separation type pharmaceutical preparation-3 (gelatin hard capsules) | |
|---|---|
| Ornoprostil-containing soft capsules | Amount (mg) |
| Components of formula liquid | |
| Ornoprostil | 0.005 |
| Tricaprylin | 50 |
| Components of capsule coating | |
| Gelatin | 35.4 |
| Concentrated glycerin | 8.2 |
| Diclofenac-containing soft capsules | |
| Components of formula liquid | |
| Diclofenac sodium | 12.5×2 |
| MCT | 37.5×2 |
| Components of capsule coating | |
| Gelatin | 35.4×2 |
| Glycerin | 8.15×2 |
| Total | 280.705 |

Preparation Example 11: Separation type preparation-4

1) Production of ornoprostil-containing liquid

To 950 g of tricaprylin, 19 mg of ornoprostil was added, followed by mixing to obtain an ornoprostil-containing liquid.

The above-described formula liquid was made into soft capsules by carrying out filling, shaping and drying in the usual method using a soft capsule coat containing gelatin, concentrated glycerin and diclofenac sodium, thereby producing oblong type (breadth 6.7 mm, length 19.4 mm) soft capsules (a total of 1,200 capsules).

| Formula of separation type pharmaceutical preparation-4 (soft capsules) | |
|---|---|
| Components of formula liquid | Amount (mg) |
| Ornoprostil | 0.005 |
| Tricaprylin | 300 |
| Components of soft capsule coating | |
| Gelatin | 185 |
| Concentrated glycerin | 55 |
| Diclofenac sodium | 25 |
| Total | 565.005 |

Preparation Example 12: Separation type preparation-5

1) Production of ornoprostil-containing seamless capsules (Composition 1)

In 250 g of tricaprylin, 50 mg of ornoprostil was dissolved, and seamless capsules (100 g in total; 1 capsule weight about 0.83 mg) having an outer diameter of 1 mm were prepared by a conventional method using a coating containing 54 g of gelatin and 6 g of concentrated glycerin.

2) Production of diclofenac sodium granules (Composition 2)

According to the usual method, 25 g of diclofenac sodium and 44 g of lactose, 22 g of corn starch, 45 g of Avicel and 4 g of Colidon 25 were granulated, dried and screened to obtain granules having an average particle size of 420 μm.

3) Production of tablets

After 30.6 mg of the above-described seamless capsules (Composition 1) were mixed with 35 mg of lactose, 15 mg of corn starch and 0.3 mg of magnesium stearate, the mixture was made into tablets to obtain 1,000 core tablets containing 0.005 mg of ornoprostil per one tablet. Separately, 100 g of diclofenac sodium granules for outer shell use (Composition 2) and 0.71 g of magnesium stearate were mixed and used as a granular powder for outer shell use. The granular powder for outer shell use was coated on the periphery of the core tablets in an amount of 141 mg per core tablet to obtain dry-coated tablets of 7 mm in outer diameter (a total of 500 tablets).

| Formula of separation type pharmaceutical preparation-5 (dry-coated tablets) | |
|---|---|
| For core tablet use | Amount (mg) |
| Seamless capsule | |
| Components of formula liquid | |
| Ornoprostil | 0.005 |
| Tricaprylin | 25.0 |
| Components of capsule coating | |
| Gelatin | 5 |
| Concentrated glycerin | 0.56 |
| Additive agents for core tablet use | |
| Lactose | 35 |
| Corn starch | 15 |

(continued)

| Formula of separation type pharmaceutical preparation-5 (dry-coated tablets) | |
|---|---|
| For core tablet use | Amount (mg) |
| Additive agents for core tablet use | |
| Magnesium stearate | 0.3 |
| For outer shell use | |
| Granules | |
| Diclofenac sodium | 25 |
| Lactose | 44 |
| Corn starch | 22 |
| Avicel | 45 |
| Colidon 25 | 4 |
| Additive agent for outer shell use | |
| Magnesium stearate | 1 |
| Total | 221.865 |

Preparation Example 13: Separation type preparation-6

Gelatin hard capsules (a total of 500 capsules) of the following formula were produced in the usual method by filling No. 4 gelatin hard capsules with 30.6 mg of the seamless capsules (Composition 1) produced in Preparation Example 12 and 140 mg of the granules (Composition 2) produced in Preparation Example 12.

| Formula of separation type pharmaceutical preparation-6 (gelatin hard capsules) | |
|---|---|
| Seamless capsule | Amount (mg) |
| Components of formula liquid | |
| Ornoprostil | 0.005 |
| Tricaprylin | 25.0 |
| Components of capsule coating | |
| Gelatin | 5 |
| Concentrated glycerin | 0.56 |
| Diclofenac-containing soft capsules | |
| Diclofenac sodium | 25 |
| Lactose | 44 |
| Corn starch | 22 |
| Avicel | 45 |
| Colidon 25 | 4 |
| Total | 170.565 |

Test Example 1: Effect of ornoprostil on NSAID-induced gastric ulcer

(1) Effect on NSAID-induced gastric ulcer (non-inflammation model)

Lew/Crj line male rats fasted for 16 to 24 hours under free water taking were checked for their body weights and then divided into groups in such a manner that average of body weights in each group became almost uniform. Each sample liquid (an OU-un-containing solution or OU solution was administered just before the administration of each NSAID suspension (to be called "OU-un-containing solution administration group" and "OU solution administration group", respectively)) was orally administered, and then each rat was sacrificed by cervical vertebrae dislocation 4 hours thereafter to extract the stomach. Also, in order to facilitate observation of gastric mucosal disorders, 0.5 ml of physiological saline containing 4% brilliant blue 6B was injected from caudal vein 3.5 hours after the sample liquid administration. The extracted stomach was filled with physiological saline and then fixed by soaking it in 10% neutrally buffered formalin. After the fixation, this was incised along the greater curvature and lightly washed in physiological saline, and then the presence of ulcer development was observed and length of the ulcer was measured. Ulcer inhibition ratio (ratio of the length of ulcer in the OU solution administration group to the length of ulcer in the OU-un-containing solution administration group) was calculated. Also, the following media were used for the preparation of sample liquids.

· Each NSAID suspension:

0.5% methyl cellulose suspension,

· OU-un-containing solution or OU solution:

phosphate buffer (pH 7.4) containing 0.5% ethanol.

As a result of the preliminary test for setting the NSAID dose (as the sample liquid, each NSAID suspension alone was administered), it was found that Di and Ac were 30 mg/kg and In was 20 mg/kg as a dose capable of inducing similar degree of ulcer between respective NSAID administration groups, so that these doses were used in this test and subsequent tests.

(2) Analgesic effect in inflammation model, effect on NSAID-induced gastric ulcer

After measuring body weight of Lew/Crj line male rats, *Mycobacterium butyricum* cells suspended in liquid paraffin was administered by intracutaneous injection into the sole of left-side hind leg of each animal, under ether anesthesia at a dose of 0.1 ml/animal, thereby inducing inflammation. Also, this day was used as the 1st day of the inflammation induction treatment. On the 21st day of the inflammation induction treatment, body weight was measured, the ankle joint of right-side hind leg was stimulated by bending and stretching it 5 times at intervals of 4 to 5 seconds, and rats which showed squeaking reaction in all of the 5 times of stimulation were selected and divided into groups in such a manner that average body weight in each group became almost uniform. Also, they were fasted for 16 to 24 hours under free water taking from the previous day of the measurement. Each sample liquid (an OU-uncontaining solution or OU solution was administered just before the administration of each NSAID suspension) was orally administered, and the squeaking reaction was observed at intervals of 1 hour during a period of from 1 hour to 4 hours after the administration. The same stimulation was added at the time of each measurement, and an animal which did not squeak by all of the 5 trial was judged squeaking reaction negative. A rat which was squeaking reaction-negative once or more by the 4 times of judgment after the sample liquid administration was judged analgesic effect-positive, and efficacy ratio (the number of animals having analgesic effect/the number of tested animals) was calculated. In addition, ulcer inhibition ratio was calculated in the same manner as described in (1). Measured results of (1) and (2) are shown in Table 1.

Table 1: Antiulcer effect and analgesic effect by simultaneous administration of NSAID and OU
(rat oral administration, non-inflammation model and inflammation model)

| Drug (dose) | (1) Non-inflammation model | (2) Inflammation induced model | |
|---|---|---|---|
| | Ulcer inhibition ratio (%) | Ulcer inhibition ratio (%) | Analgesic effective ratio (the number of cases) |
| Invention | Di (30mg/kg) + OU (3μg/kg) | 67.30 | 45.20 | 8/10 |
| Comparison 1 | In (20mg/kg) + OU (3μg/kg) | 15.80 | 29.40 | 5/10 |
| Comparison 2 | Ac (30mg/kg) + OU (3μg/kg) | 37.70 | 0 | 8/10 |

It can be understood from Table 1 that, in comparison with the combinations of In + OU administration group (Comparison 1) and Ac + OU administration group (Comparison 2), the combination of Di + OU administration group (Invention) is the most suited combination, because its analgesic effect (main activity) and ulcer inhibition effect (side effect reducing activity) are both strong.

In addition, since the analgesic effect effective ratio in each NSAID was equivalent in the OU administered group and OU un-administered group, the OU administration did not exert influence upon the analgesic effect of NSAID

Test Example 2: Comparison of the inhibitory effects of ornoprostil and misoprostol on diclofenac sodium-induced gastric ulcer

Lew/Crj line male rats fasted for 16 to 24 hours under free water taking were checked for their body weights and then divided into groups in such a manner that average of body weights in each group became almost uniform. Each sample liquid (OU-un-containing solution or OU solution, MI-un-containing solution or MI solution, was administered just before the administration of 30 mg/kg Di suspension) was orally administered, and each rat was sacrificed by cervical vertebrae dislocation 4 hours thereafter to extract the stomach. Thereafter, according to Test Example 1, ulcer inhibition ratio (ratio of the length of ulcer in each PG (OU, MI) solution administration group to the length of ulcer in each PG (OU, MI)-un-containing solution administration group) was calculated. In this case, the following media were used for the preparation of sample liquids. The results are shown in Table 2.

·   Di suspension:

    0.5% methyl cellulose suspension,

·   each PG (OU, MI)-un-containing solution or OU and MI solutions:

    phosphate buffer (pH 7.4) containing 0.5% ethanol.

Table 2:

| Inhibitory effect of ornoprostil and misoprostol on diclofenac sodium-induced gastric ulcer | | |
|---|---|---|
| Drugs (dose) | | Ulcer inhibition ratio (%) |
| Invention | Di (30 mg/kg) + OU (3 µg/kg) | 67.3 |
| Comparative | Di (30 mg/kg) + MI (30 µg/kg) | 56.0 |

As shown in Table 2, ornoprostil showed 10 times or more higher antiulcer activity than that of misoprostol on diclofenac sodium-induced gastric ulcer.

Test Example 3: Diarrhea-inducing activity (rats, oral administration)

The diarrhea-inducing activity was 400 µg/kg (0 case/5 cases) or more by ornoprostil and 50 and 100 µg/kg (0 case/5 cases and 1 case/5 cases, respectively) by misoprostol, respectively, so that the safety margin (maximum nontoxic amount) of ornoprostil was 8 times or more higher than that of misoprostol (the value in parentheses means the number of diarrhea-induced animals/the number of tested animals).

Accordingly, the Di + OU administered group showed 80 times or more superior usefulness in comparison with the Di + MI administered group.

From the results of the above tests 1 to 3, it can be seen that a combination of Di and OU is the most suitable as the combination of NSAID and PG.

Test Example 4: Administration timing and antiulcer effect of diclofenac sodium and ornoprostil

Ulcer inhibitory effects (ulcer developing ratio = the number of ulcer-developed animals/the number of animals tested, and ulcer inhibition ratio) were measured when ornoprostil solution was administered before the administration of diclofenac sodium suspension, simultaneously with the administration of diclofenac sodium suspension (just before the administration) or after the administration of diclofenac sodium suspension. The results are shown in Table 3.

Table 3:

| Relationship between the timing of ornoprostil administration and the inhibitory effect on diclofenac sodium-induced gastric ulcer (rats, oral administration) | | | |
|---|---|---|---|
| Timing of OU administration | Ulcer development ratio (the number of cases) | Ulcer inhibition ratio (%) | Antiulcer factor* |
| 60 min. before Di admin. | 7/8 | 85.1 | 97.3 |
| 30 min. before Di admin. | 7/8 | 49.2 | 56.2 |
| 10 min. before Di admin. | 5/8 | 78.2 | 125.1 |
| 5 min. before Di admin. | 6/8 | 75.7 | 100.9 |
| 0 min. before Di admin. | 7/8 | 76.0 | 86.9 |
| 5 min. after Di admin. | 7/8 | 6.2 | 7.1 |
| 10 min. after Di admin. | 8/8 | 28.8 | 28.8 |
| 30 min. after Di admin. | 7/8 | 14.1 | 16.1 |
| 60 min. after Di admin. | 6/8 | 3.1 | 4.1 |

*: Antiulcer factor = ulcer inhibition ratio/ulcer development ratio

Regarding the ulcer inhibitory effect, simultaneously with Di or before Di administration, particularly from 10 minutes to 5 minutes before Di administration (antiulcer factor as the index) was effective as the timing of OU administration, but the effect was attenuated by after administration.

Test Example 5: Antiphlogistic effect (blood concentration of diclofenac sodium) of the pharmaceutical preparation of the present invention

After 1 hour of feeding, each of the Di-containing pharmaceutical preparation samples described in the following (the number which corresponds to 75 mg as the dose of Di) was orally administered by force to female beagle (10 to 15 months of age, a total of 5 animals) which had been fed with a solid feed (DS-5 (trade name); Oriental Yeast, 200 g/once/day) under free water taking. Just after that, water (10 ml) was orally administered by force using a gastric catheter. Blood samples were collected from a cephalic vein before administration of the sample and 0.5, 1, 2, 3, 4, 6 and 24 hours after the administration. The collected blood samples were centrifuged (4°C, 3,000 rpm, 15 minutes) to obtain plasmas. The thus obtained plasmas were measured for the concentration of Di.

Thereafter, the same test operation was carried out twice (3 times in total) each week using respective test animals by changing Di-containing pharmaceutical preparation samples (cf. the following). Based on the changes in the measured blood Di concentration, $C_{max}$ (maximum blood concentration), $T_{max}$ (time from the commencement of administration until reaching maximum blood concentration) and AUC (area under curve) (average value) were calculated. The results are shown in Table 4.

Di-containing pharmaceutical preparation samples

· Preparation produced in Preparation Example 1:

   a Di + OU combination agent (a combination agent of Di (25 mg) + OU (5 μg))

· Comparative preparation 1:

   a Di single preparation (Voltaren (trade name), a single preparation of Di (25 mg))

· Comparative preparation 2:

   a Di + MI combination agent (Arthrotec Tablet: trade name), a combination agent of Di (75 mg) + MI (200 μg))

Table 4:

| Antiphlogistic effect of the pharmaceutical preparation of the present invention (blood concentration of diclofenac sodium) (n = 5) | | | |
|---|---|---|---|
| Administered preparation (Administered number) | $C_{max}$ (μg/ml) | $T_{max}$ (hr) | AUC (μg·hr/ml) |
| Preparation produced in Preparation Example 1 (3 capsules) | 14.0 | 1.5 | 52.8 |
| Comparative preparation 1 (3 tablets) | 11.2 | 5.8 | 52.6 |
| Comparative preparation 2 (1 tablet) | 6.7 | 19.4 | 21.1 |

As shown in Table 4, the preparation of the present invention has a $C_{max}$ value of equal to or larger than that of Comparative preparations 1 ad 2, so that it can be sufficiently predicted that the former has equivalent or more effects (antipyretic, analgesic, antiphlogistic). Also, since the preparation of the present invention has quicker $T_{max}$ than that of Comparative preparations 1 and 2, it is expected that the former has fast-acting property. In addition, it was found that the preparation of the present invention and Comparative preparation 1 are almost the same in terms of AUC.

It can be understood from the above that the combination agent of the present invention comprising diclofenac sodium and ornoprostil has effects (antipyretic, analgesic and antiphlogistic effects) and fast-acting property which are equal to or larger than those of the commercially available diclofenac sodium single preparation and the commercially available combination agent of diclofenac sodium and misoprostol,

Test Example 6: Effect of ornoprostil on nephropathy

SPF/Crj line male rats (9 week-old) were measured for their body weight and then divided into groups in such a manner that average of body weights of respective groups became almost uniform. A 50% glycerol solution (medium: physiological saline) (10 ml/kg) was administered by subcutaneous injection to each rat which had been abstained from water for 48 hours (± 2 hours) but under feeding, and about 1.5 ml of blood was collected 48 hours thereafter from jugular vein under ether anesthesia. Ornoprostil (medium: 0.4% Tween 80 solution containing 1% anhydrous ethanol) was orally administered just before the glycerol administration. Water was provided 2 hours after the glycerol administration. The thus collected blood was centrifuged (about 4°C, 3,000 rpm, 15 minutes) to obtain serum. The thus obtained serum was subjected to the measurement of urine nitrogen (to be referred to as UN) (urease-GIDH method) and creatinine (to be referred to as CRE) (creatininase-F-DAOS method) using a biochemical automatic analyzer (AU 400 (trade name), Olympus Optical). The results are shown in Table 5.

Table 5.

| Nephropathy induction-inhibiting effect of ornoprostil | | | |
|---|---|---|---|
| Test group | Dose | UN (mg/dL) | CRE (mg/dL) |
| Normal control | Medium*(subcutaneous) + medium** | 18.8 | 0.19 |
| Inflamed control | GC (subcutaneous) + medium** | 163.7 | 2.74 |
| Invention | GC (subcutaneous) + OU (3 μg) | 89.7 | 1.38 |
|  | GC (subcutaneous) + OU (10 μg) | 79.9 | 1.03 |
| *: Physiological saline, | | | |

**: 0.4% Tween 80 solution containing 1% ethanol

From Table 5, it can be seen that ornoprostil significantly inhibits an ischemic renal insufficiency model, glycerol-induced nephropathy (increase of UN value and CRE value in serum), and therefore is effective for nephropathy. In addition, its dose is the same amount of the dose that showed the gastric ulcer-inhibiting effect accompanied by NSAID shown in Test Example 1. Accordingly, it can be expected that the combination agent of ornoprostil and diclofenac sodium can reduce worsening of nephropathy possessed by diclofenac. Also, the nephropathy-inhibiting activity is an effect which cannot be found by acid secretion inhibitors and/or protective factor improvers.

Test Example 7: Effect of dosage forms on the stability of ornoprostil at the time of their production

Formation ratio of ornoprostil degradation product (11-deoxy-$\Delta^{10}$ form: 17S,20-dimethyl-6-oxo-prostaglandin Al methyl ester) at the time of the production of Preparation Examples 2 to 5 was analyzed by high performance liquid chromatography (HPLC).

The formation ratio of degradation product was calculated by defining the total of ornoprostil and degradation product as 100. The results of analysis are shown in Table 6.

Table 6:

| Formation ratio (%) of ornoprostil degradation product | | | | |
|---|---|---|---|---|
| | Preparation Example 2 | Preparation Example 3 | Preparation Example 4 | Preparation Example 5 |
| Just after production | 19.4 | 0.0 | 0.0 | 0.5 |

While the soft capsule preparation shown by Preparation Example 2 showed a degradation product formation ratio of about 20% just after its production, the hard capsule preparations of Preparation Examples 3 to 5 hardly produced the 11-deoxy-$\Delta^{10}$ form.

Test Example 8: Influence of capsule materials upon the stability of ornoprostil during preservation

Each of the preparations of Preparation Examples 3 to 5 was packed in a glass bottle, and the packed bottle was preserved at 60°C in a stability tester. Samples were collected after 2 weeks and 4 weeks, and the formation ratio of degradation product (11-deoxy-$\Delta^{10}$ form) was analyzed by HPLC. Preparation of samples to be analyzed and the HPLC conditions are as described in Test Example 7. The results of analysis are shown in Table 7.

Table 7:

| Formation ratio (%) of ornoprostil degradation product | | | | |
|---|---|---|---|---|
| | | Preparation Example 3 | Preparation Example 4 | Preparation Example 5 |
| Periodical stability | 60°C, 2 weeks | 0.0 | 0.0 | 0.3 |
| | 60°C 4 weeks | 41.8 | 0.5 | 1.9 |

While a degradation formation ratio of about 40% was found under a condition of 60°C14 weeks in the hard capsule shown by Preparation Example 3, the HPMC hard capsules shown by Preparation Examples 4 and 5 hardly formed the 11-deoxy-$\Delta^{10}$ form.

Test Example 9: Stability of ornoprostil in separation type pharmaceutical preparation

Using Preparation Example 8 as a typical example of the separation type pharmaceutical preparation, periodical stability of ornoprostil in the separation type pharmaceutical preparation was evaluated by comparing with a mixture type pharmaceutical preparation (Preparation Example 4). As the formation ratio of degradation products, degradation products of ornoprostil (8-iso form: 17S,20-dimethyl-6-oxo-8-isoprostaglandin E1 methyl ester; 11-deoxy-$\Delta^{10}$ form: 17S,20-dimethyl-6-oxo-prostaglandin A1 methyl ester) were analyzed using high performance liquid chromatography (HPLC).

The formation ratio of degradation products was calculated by defining the total of ornoprostil and each degradation product as 100. The results of analysis are shown in Table 8.

Table 8:

| Degradation formation ratio (%) of 8-iso form and 11-deoxy form at 60°C | | | | | |
|---|---|---|---|---|---|
| Preparation Example 4 | | | Preparation Example 8 | | |
| Days | 8-Iso form | 11-Deoxy form | Days | 8-Iso form | 11-Deoxy form |
| 2 | 7.4 | 0.0 | - | - | - |
| 5 | 12.4 | 0.0 | 7 | 0.0 | 0.0 |
| 12 | 16.5 | 0.0 | 14 | 0.0 | 0.0 |
| 27 | 18.0 | 0.0 | 28 | 0.0 | 0.0 |

While the mixture type pharmaceutical preparation shown by Preparation Example 4 periodically formed the 8-iso form as a degradation product at 60°C, the separation type pharmaceutical preparation of Preparation Example 8 was markedly stable without forming the 8-iso form and 11-deoxy form.

Test Example 10: Side effects (gastric and small intestine ulcers, diarrhea and vomiting)-reducing effect of the preparation of the present invention

After measuring their body weights, male beagles (14 to 19 month-old) which had been reared under free water taking were divided into groups in such a manner that average of body weights in respective groups becomes almost uniform (6 to 8 animals in each group). Each of Di-containing pharmaceutical preparation samples (the number which corresponds to 225 mg as the dose of Di) was orally administered to the test animals by force. General conditions (vomiting and diarrhea) were observed during 6 hours after the administration.

On the next day of administration, 4% brilliant blue (20 ml) was administered by intravenous injection, and after 2 hours or more of leaving alone, pentobarbital sodium was administered to each beagle by intravenous injection (25 mg/kg), and then a canula was inserted into the carotid artery under anesthesia to effect bloodletting death. The stomach was taken out. After incising the stomach in such a manner that the gastric mucosa can be observed, the ulcer-generated parts were counted, and length and breadth of each ulcer were measured using calipers to calculate ulcer area ($mm^2$). In addition, Voltaren tablet and Arthrotec tablet were used as comparative examples. The presence or absence of vomiting or diarrhea during 6 hours after the administration and generation ratio of ulcer after 1 day of the administration are shown in the following Table 9.

Di-containing pharmaceutical preparation samples

· Preparation produced in Preparation Example 7:

   a Di + OU combination agent (a combination agent of Di (25 mg) + OU (5 μg))

· Comparative preparation 1:

   a Di single preparation (Voltaren Tablet (trade name), a single preparation of Di (25 mg))

· Comparative preparation 2:

   a Di + MI combination agent (Arthrotec Tablet: trade name), a combination agent of Di (75 mg) + MI (200 μg))

Table 9:

| Side effects (gastric ulcer, diarrhea and vomiting)- reducing effect of the preparation of the present invention | | | |
|---|---|---|---|
| Administered preparation (administered numbers) | Number of generated vomiting or diarrhea | Number of gastric mucosa ulcer | Area of gastric mucosa ulcer ($mm^2$) |
| Preparation produced in Preparation Example 7 (9 capsules) | 1/6 | 11.3 ± 3.9# | 40.16 ± 10.40# |
| Comparative preparation 1 (9 tablets) | 0/8 | 46.4 ± 9.2 | 144.14 ± 21.40 |
| Comparative preparation 2 (3 tablets) | 4/6* | 87.5 ± 40.8 | 353.68 ± 132.70 |

* $p < 0.05$ vs. Comparative preparation 1 (9 tablets) (Fisher test)
# $p < 0.05$ vs. Comparative preparation 1 (9 tablets) (nonparametric Dunnet multiple comparison test)

When degree of the gastric mucosa ulcer by the preparation produced in Preparation Example 7 was compared in terms of the number of gastric mucosa ulcers and the area of gastric mucosa ulcers, significant reduction was found in both cases by the preparation produced in Preparation Example 7, in comparison with the Comparative preparation 1. However, in comparison with the Comparative preparation 1, onset of these gastric ulcers showed an increasing tendency on the contrary by the Comparative preparation 2. On the other hand, ulcer of small intestine mucosa was not observed by both of the preparation produced in Preparation Example 7 and Comparative preparation 1, but the ulcer of small intestine was found in 1 case of 6 cases by the Comparative preparation 2.

Although generation of vomiting or diarrhea was not found in all of the cases on the administered day in the case of the Comparative preparation 1, vomiting or diarrhea was found in 4 cases among the 6 cases by the Comparative preparation 2, thus showing a significant difference from the Comparative preparation 1 in terms of their onset. On the other hand, diarrhea was found in only 1 case among the 6 cases in the case of the preparation produced in Preparation Example 7.

Diclofenac concentrations in blood by respective preparations were measured to carry out comparative evaluation. As a result, the preparation produced in Preparation Example 7 showed quick appearance of diclofenac sodium in blood, and the $T_{max}$ value also showed a quickening tendency, in comparison with the Comparative preparation 1, but the values of $C_{max}$ and AUC were almost the same as those of the Comparative preparation 1. On the other hand, in the case of the Comparative preparation 2, appearance of dielofenac sodium in blood was delayed, but the values of $C_{max}$ and AUC were almost the same as those of the Comparative preparation 1.

Test Example 11: Dissolution test of the pharmaceutical preparation of the present invention

Dissolution test of the active ingredients (omoprostil and diclofenac sodium) of the pharmaceutical preparation produced in Preparation Example 8 was carried out under the following conditions.

Apparatus: second method (paddle method) of the dissolution test method in general test methods, The Pharmacopoeia of Japan,
Test liquid volume: 900 ml,
Temperature; $37 \pm 0.5°C$,
Speed of rotation: 50 rpm, and
Test liquid: the first liquid (pH 1.2) of disintegration test, The Pharmacopoeia of Japan.

After commencement of the dissolution test, 20 ml of eluate was collected at each predetermined time, and the same volume of test liquid heated to 37°C was immediately added thereto to compensate for the lost portion. The eluate was filtered through a membrane filter having a pore size of 0.5 µm or less, the first 10 ml of the filtrate was discarded, and the next filtrate was used as the test liquid.

Diclofenac sodium was measured by spectrophotometry for ultraviolet and visible region, and ornoprostil was measured by liquid chromatography. As a result, it was confirmed that dissolution of ornoprostil is quicker than that of diclofenac sodium.

**Claims**

1. An agent for reducing side effects of diclofenac or a salt thereof, which comprises ornoprostil as the active ingredient.

2. The agent for reducing side effects according to claim 1, wherein the side effects are digestive disorders.

3. The agent for reducing side effects according to claim 1, which is a combination agent comprising an effective amount of ornoprostil and an effective amount of diclofenac or a salt thereof.

4. The agent for reducing side effects according to claim 3, wherein the effective amount of ornoprostil is a biological catalytic amount.

5. The agent for reducing side effects according to claim 4, wherein the biological catalytic amount of ornoprostil is from 1/100,000 to 1/1,000 based on 1 part by weight of diclofenac or a salt thereof.

6. The agent for reducing side effects according to claim 3, which comprises about 25 mg of diclofenac sodium and about 5 µg of ornoprostil.

7. The agent for reducing side effects according to claim 3, which is a single solid preparation which comprises at least one small soft capsule preparation (A) comprising ornoprostil and an oily solution base, and a pharmaceutical composition (D-1) comprising diclofenac or a salt thereof and an excipient.

8. The agent for reducing side effects according to claim 7, wherein the solid preparation is a hard capsule preparation.

9. The agent for reducing side effects according to claim 8, which comprises from 40 to 95% by weight of the oily solution base based on 100% by weight of the small soft capsule preparation (A), and from 20 to 95% by weight of the excipient based on 100% by weight of the pharmaceutical composition (D-1).

10. The agent for reducing side effects according to claim 8, wherein the number of the small soft capsule preparation (A) is from 1 to 400.

11. The agent for reducing side effects according to claim 8, wherein the number of the small soft capsule preparation (A) is 1 or 2.

12. The agent for reducing side effects according to claim 10, wherein the small soft capsule preparation (A) has an outer diameter of from 0.1 mm to 2 mm.

13. The agent for reducing side effects according to claim 11, wherein the small soft capsule preparation (A) has an outer diameter of from 2 mm to 6 mm.

14. The agent for reducing side effects according to claim 7, wherein the oily solution base is middle chain fatty acid triglyceride.

15. The agent for reducing side effects according to claim 14, wherein the middle chain fatty acid triglyceride is tricaprylin.

16. The agent for reducing side effects according to claim 7, wherein the excipient is one or at least two substances selected from the group consisting of saccharides, corn starch, and crystalline cellulose.

17. The agent for reducing side effects according to claim 7, wherein the pharmaceutical composition (D-1) is in the form of granules.

18. The agent for reducing side effects according to claim 17, wherein the granules have an average particle size of from about 200 μm to about 600 μm.

19. The agent for reducing side effects according to claim 7, wherein the solid preparation is in the form of tablets.

20. The agent for reducing side effects according to claim 3, which is a single solid preparation which comprises at least one small soft capsule preparation (A) comprising ornoprostil and an oily solution base, and at least one small soft capsule preparation (C) comprising diclofenac or a salt thereof and an oily solution base.

21. The agent for reducing side effects according to claim 20, wherein the solid preparation is in the form of hard capsules.

22. The agent for reducing side effects according to claim 20, wherein the oily solution base in the soft capsule preparation (A) is tricaprylin, and the oily solution base in the soft capsule preparation (C) is middle chain fatty acid triglyceride,

23. The agent for reducing side effects according to claim 20, wherein the solid preparation is in the form of tablets.

24. The agent for reducing side effects according to claim 3, which is a single solid preparation which comprises a pharmaceutical composition (B) comprising ornoprostil and an oily solution base, and at least one small soft capsule preparation (C) comprising diclofenac or a salt thereof and an oily solution base.

25. The agent for reducing side effects according to claim 24, wherein the solid preparation is in the form of hard capsules.

26. The agent for reducing side effects according to claim 24, wherein the solid preparation is in the form of soft capsules.

27. The agent for reducing side effects according to claim 3, which is a single solid preparation which comprises at least one small soft capsule preparation (A) comprising ornoprostil and an oily solution base, and a pharmaceutical composition (D-2) comprising diclofenac or a salt thereof and an oily solution base.

28. The agent for reducing side effects according to claim 27, wherein the solid preparation is in the form of soft capsules.

29. The agent for reducing side effects according to claim 27, wherein the solid preparation is in the form of hard capsules.

30. The agent for reducing side effects according to claim 3, which is a soft capsule preparation wherein a pharmaceutical composition (B) comprising the ornoprostil and an oily solution base is coated with a soft capsule coat comprising the diclofenac or a salt thereof.

31. The agent for reducing side effects according to claim 1, which is a soft capsule preparation wherein a pharmaceutical composition (D-2) comprising the diclofenac or a salt thereof and an oily solution base is coated with a soft capsule coat comprising the ornoprostil.

32. A side effect-reducing hard capsule agent for reducing digestive disorders of diclofenac or a salt thereof, which is a hard capsule preparation which comprises at least one small soft capsule preparation (A-1) comprising ornoprostil and from 40 to 95% by weight of an oily solution base, and a pharmaceutical preparation (D-3) comprising diclofenac or a salt thereof and from 20 to 95% by weight of an excipient.

33. The hard capsule agent for reducing side effects according to claim 32, wherein the oily solution base is tricaprylin, and the excipient is one or at least two substances selected from the group consisting of saccharides, corn starch, and crystalline cellulose.

## INTERNATIONAL SEARCH REPORT

| International application No. |
| --- |
| PCT/JP03/10562 |

A. CLASSIFICATION OF SUBJECT MATTER
Int.Cl⁷ A61K31/196, 31/5575, 9/48, 47/14, 47/38, A61P1/00, 13/12, 29/00, 43/00

According to International Patent Classification (IPC) or to both national classification and IPC

B. FIELDS SEARCHED

Minimum documentation searched (classification system followed by classification symbols)
Int.Cl⁷ A61K31/196, 31/5575, 9/48, 47/14, 47/38, A61P1/00, 13/12, 29/00, 43/00

Documentation searched other than minimum documentation to the extent that such documents are included in the fields searched

Electronic data base consulted during the international search (name of data base and, where practicable, search terms used)
CAPLUS(STN), EMBASE(STN), MEDLINE(STN), BIOSIS(STN)

C. DOCUMENTS CONSIDERED TO BE RELEVANT

| Category* | Citation of document, with indication, where appropriate, of the relevant passages | Relevant to claim No. |
| --- | --- | --- |
| P,X | WO 02/066030 A1 (ONO PHARMACEUTICAL CO., LTD.), 29 August, 2002 (29.08.02), Claims 4, 5, 21, 22 (Family: none) | 1-33 |
| X | ARES J.J. et al., Gastroprotective agents for the prevention of NSAID-induced gastropathy, Current Pharmaceutical Design, 1998, Vol.4, No.1, pages 17 to 36; especially left column of page 20 | 1-33 |
| A | PALLER Mark S. et al., Prostaglandins protect kidneys against ischemic and toxic injury by a cellular effect, Kidney International, 1993, Vol.42, pages 1345 to 1354 | 1-33 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 05 november, 2003 (05.11.03) | 18 November, 2003 (18.11.03) |

| Name and mailing address of the ISA/ | Authorized officer |
| --- | --- |
| Japanese Patent Office | |
| Facsimile No. | Telephone No. |

Form PCT/ISA/210 (second sheet) (July 1998)

**INTERNATIONAL SEARCH REPORT**

International application No.

PCT/JP03/10562

C (Continuation). DOCUMENTS CONSIDERED TO BE RELEVANT

| Category* | Citation of document, with indication, where appropriate, of the relevant passages | Relevant to claim No. |
|---|---|---|
| A | WO 96/03992 A1 (Loyola University of Chicago), 15 February, 1996 (15.02.96), & EP 767665 A1 & JP 10-503522 A | 1-33 |
| A | EP 1068867 A2 (SHERMAN BERNARD CHARLES), 17 January, 2001 (17.01.01), & JP 2001-39876 A | 1-33 |

Form PCT/ISA/210 (continuation of second sheet) (July 1998)